# EUROPEAN PATENT APPLICATION

(11) **EP 1 616 871 A1**
(43) Date of publication of application: **18.01.2006**
(21) Application number: 04728406.2
(22) Date of filing: 20.04.2004
(51) Int. Cl.: C07D 487/22, A61K 31/409, A61K 31/555, A61P 29/00, A61P 35/00, G01N 30/88

(54) **PORPHYRIN COMPOUND CONTAINING BIOTINYL GROUP AND USE THEREOF**

(30) Priority: 21.04.2003 JP 2003116232
(71) Applicant: RIKEN, Wako-shi, Saitama 351-0198 (JP)
(72) Inventor: ISOGAI, Yasuhiro, 1780061 (JP); ISHIDA, Manabu, 3510015 (JP)
(74) Representative: White, Nina Louise
(86) International application number: PCT/JP2004/005645
(87) International publication number: WO 2004/094432

(57) **Abstract**

The present invention provides a porphyrin compound containing a biotinyl group represented by Formula (I):
Por-A-Bi

wherein Por represents a porphyrin residue optionally forming a metal complex; Bi represents an optionally substituted biotinyl group; and A represents a C₁-C₂₀ hydrocarbyl group, or a heterohydrocarbyl group having 1-5 heteroatoms selected from a group consisting of oxygen, sulfur, and nitrogen, and having 1-20 atoms in total; a method for purifying hemoprotein which use the porphyrin compound; a hemoprotein labeling reagent; a diagnostic agent for hemoprotein associated diseases which use the porphyrin compound; and a therapeutic agent for photodynamic therapy.

## Description

### TECHNICAL FIELD

The present invention relates to a porphyrin compound containing a biotinyl group, and more particularly, it relates to a porphyrin compound containing a biotinyl group that can purify small amounts of hemoprotein in the living body rapidly and simply. The present invention also relates to a purification method for hemoprotein and apparatus therefor utilizing such a porphyrin compound containing a biotinyl group; a labeling reagent for hemoprotein; a method for the detection of hemoprotein and a diagnostic agent for hemoprotein-associated diseases utilizing that reagent; and a therapeutic drug for photodynamic therapy that contains the above porphyrin compound containing a biotinyl group.

### BACKGROUND ART

Iron protoporphyrin IX, which is called "protoheme" or simply "heme," performs various roles as an active center for a plurality of proteins such as an enzyme and oxygen carrier, and also a biosensor (see A. Messerschmidt, R. Huber, T. Poulos, and K. Wieghardt (Eds), Handbook of Metalloproteins Vol. 1, John Wiley & Sons, New York, 2001, etc.). Therefore, detecting and isolating hemoprotein are important for research on these physiological functions.

In prior art hemin agarose has been used for the purification of hemoprotein as a carrier in affinity chromatography (Tsutsui & Mueller, Analytical Biochemistry 121, 244-250, 1982: non-patent document 1). However, a problem that cannot be ignored in this prior art method is the non-specific binding between proteins and the agarose that binds to the hemin. Moreover, because of the large particle size of agarose, its protein binding capacity per volume is small, and the spectroscopic detection of its specific binding with the hemoprotein is extremely difficult. In addition, hemin agarose has a shortcoming because it cannot be used for the labeling of hemoprotein.

On the other hand, photodynamic therapy (PDT) for treating diseases such as malignant tumors and rheumatoid arthritis has recently been developed in which a photoactive compound such as a porphyrin is administered to the patient and the treatment site is irradiated with light to activate the porphyrin (Japanese Patent Application Laid-open No. H 10-508577). However, therapeutic drugs for PDT that can efficiently supply the photoactive compound to the treatment site have still not been discovered.

Because of these circumstances, it would be desireble if there were provided a hemoprotein purification method that can perform the purification of hemoprotein simply and rapidly. It would also be desireble if there were provided a reagent that can label these proteins to investigate the behavior of hemoproteins (or hemoprotein metabolizing enzymes) in the living body. Further, it would be desireble if there were provided a therapeutic drug for more efficient photodynamic therapy.

### DISCLOSURE OF INVENTION

The present invention was created to solve the aforementioned prior art problems. The first embodiment of the present invention provides a porphyrin compound containing a biotinyl group represented by Formula (I):
Por-A-Bi

wherein Por represents a porphyrin residue optionally forming a metal complex; Bi represents an optionally substituted biotinyl group; and A represents a C₁-C₃₀ hydrocarbyl group, or a C₁-C₃₀ heterohydrocarbyl group having 1-10 heteroatoms selected from a group consisting of oxygen, sulfur, and nitrogen. Preferably, the Por is a porphyrin (heme) residue that has formed a metal complex selected from a group consisting of iron-porphyrin derivatives such as heme a, heme b (protoheme IX), heme c, variant heme c, heme d, heme d1, siroheme (Sirohaem), and heme o. More preferably, the Por is a heme b residue. Further, in another preferred embodiment the Por is a porphyrin residue selected from a group consisting of uroporphyrin-I, uroporphyrin-II, coproporphyrin-III, protoporphyrin-IX, and hematoporphyrin-IX). Preferably, the Bi is a biotinyl group.

Preferably, in the present invention the A is a straight chain or branched alkylene group of 1-20 carbon atoms, and one or more than one of the non-adjacent CH₂ groups of the alkylene group is optionally substituted by - NH- , - NH- NH- , - NHCO- , - CONH- , - N(C₁₋₃ alkyl)-, -O- , -S-, -CO-, -O-CO-, -S-CO-, -O-COO-, -CO-S-, -CO-O-, - CH(halogen)-, - CH(CN)- , - CH=CH- , - NH- NH- CO- or - CO- NH- NH-.

More preferably, in the present invention the A is selected from a group consisting of
- NH- NH-,
- NH- NH- CO- (CH₂)ₙ- NH- ,
- NH- NH- CO- (CH₂)ₙ- NH- CO- (CH₂)ₙ- NH- ,
- NH- (CH₂)ₙ- NH- ,
- NH- NH- CO- (CH₂)ₙ- NH- ,
- NH- NH- CO- (CH₂)ₙ- CO- NH- NH- ,
- NH- (CH₂)ₙ- CO- NH- NH- , and
- NH(CH₂)ₙ- CO- NH- (CH₂)ₙ- CO- NH- NH-

in these formulae each n independently represents 1-10, and preferably 3-7.

The second embodiment of the present invention provides a method for preparing the porphyrin compound containing a biotinyl group of Formula (I) above comprising a method for preparing a heme compound containing a biotinyl group that includes reacting a porphyrin optionally forming a metal complex with a compound containing a terminally aminated biotinyl group in the presence of a coupling agent.

The third embodiment of the present invention provides a hemoprotein purification method comprising a step of performing affinity chromatography using the compound containing a biotinyl group of Formula (I) above.

The fourth embodiment of the present invention provides a hemoprotein purification kit comprising the compound of Formula (1) above and carrier beads with an avidin compound bonded thereto.

The fifth embodiment of the present invention provides a hemoprotein labeling compound wherein a labeling substance is bound to the compound containing a biotinyl group of Formula (I) above.

The sixth embodiment of the present invention provides a method for detecting hemoprotein using the above labeling compound.

The seventh embodiment of the present invention provides a diagnostic agent for hemoprotein-associated diseases comprising the above labeling compound.

Finally, the eighth embodiment of the present invention provides a therapeutic drug for photodynamic therapy comprising a compound wherein Por in Formula (I) is a porphyrin residue.

The present invention relates to a compound wherein biotin, which is widely used for labeling and isolating biological polymers because of its high affinity with streptavidin, is bound to heme, which serves as a prosthetic group in many proteins. By using this molecule, the labeling of hemoprotein in the living body, isolation, and purification of small amounts thereof can each be performed rapidly in a single step. Because the porphyrin compound containing a biotin group of the present invention can be bound to various avidin derivatives after it alone binds to the protein, the problems associated with the aforementioned prior art method that uses hemin agarose can be solved.

In this description the term "porphyrin" refers to a cyclic tetrapyrrole that is a porphin derivative in which four pyrrole groups linked toghether and ring closure by four methine groups; these include, for example, uroporphyrin-I, uroporphyrin-III, coproporphyrin-III, protoporphyrin-IX, and hematoporphyrin-IX, etc. Heme is noted as a most suitable porphyrin that forms a metal complex.

In the present description the term "heme" refers to a coordination compound of porphyrin (or derivative thereof) and mainly bivalent or trivalent iron, and it is also called iron porphyrin and hematin. In the present invention no particular restriction is placed on the heme that is used and a natural heme, for example, heme a, heme b (protoheme IX), heme c, variant heme c, heme d, heme d1, siroheme (Sirohaem), and heme o can be used (see A. Messerschmidt, R. Huber, T. Poulos, and K. Wieghardt (Eds), Handbook of Metalloproteins Vol. 1, John Wiley & Sons, New York, 2001, etc.).

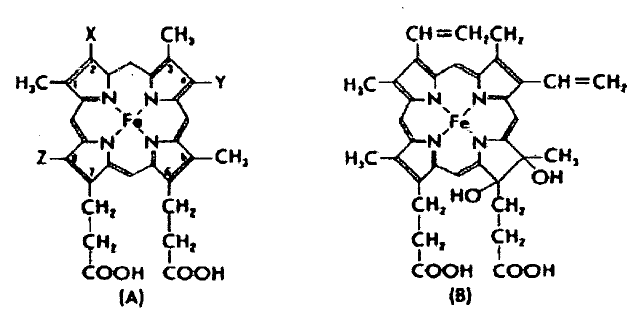

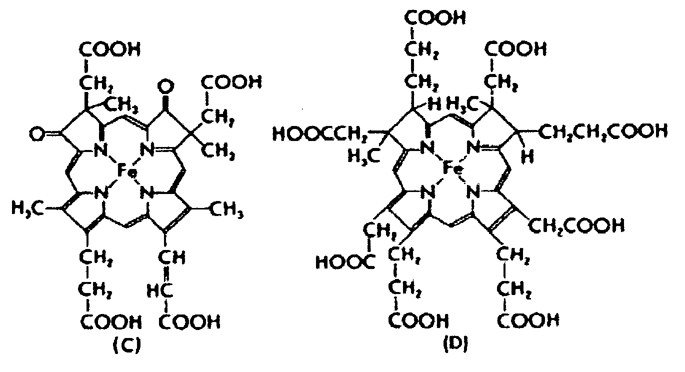

In the above formulae, X, Y, and Z each represent the moieties shown in the table below.

| | X | Y | Z |
|---|---|---|---|
| Heme b | - CH=CH₂ | - CH=CH₂ | - CH₃ |
| Heme c | - C(CH₃)H-SR^{b} | - C(CH₃)H-SR^{b} | - CH₃ |
| Variant heme c | - CH=CH₂ | - C(CH₃)H-SR^{b} | - CH₃ |
| Heme a | - CH(OH) - CH₂R'^{c} | - CH=CH₂ | - CHO |
| Heme d | Same as (B) | | |
| Heme d1 | Same as (C) | | |
| Siroheme | Same as (D) | | |
| Heme o | - CH(OH) - CH₂R'^{c} | -CH=CH₂ | - CH₃ |

| | | | |
|---|---|---|---|
| Note) SR^{b} = - CH₂- C(NH- )H- CO-, R'^{c} = - [CH₂CH=C(CH₃)CH₂]₃H | | | |

Moreover, in the present invention "heme" is not restricted to the above natural hemes, and various well-known synthetic hemes can be used. For example, such synthetic hemes are described in David Dolphin ed., The Porphyrins, Vol. 1-5, Academic Press, New York, 1978.

In the present description the term "hemoprotein" refers to a protein that can bind to a heme such as that noted above (including hemoprotein metabolic enzymes), and it includes, for example, hemoglobin, myoglobin, cytochrome, peroxidase, and catalase, etc.

In the present description the term "hydrocarbyl group" refers to an optionally saturated or unsaturated acyclic, or an optionally saturated or unsaturated cyclic, substituted or unsubstituted hydrocarbon, and if the hydrocarbon is acyclic, then it may be either straight chain or branched. Examples of C₁-C₂₀ hydrocarbons include, for example, a C₁-C₂₀ alkyl group, C₂-C₂₀ alkenyl group, C₂-C₂₀ alkynyl group, C₁-C₂₀ alkoxy group, C₁-C₂₀ acyl group, C₄-C₂₀ alkyl dienyl group, C₄-C₂₀ polyenyl group, C₆-C₁₈ aryl group, C₇-C₂₀ alkylaryl group, C₇-C₂₀ arylalkyl group, C₄-C₂₀ cycloalkyl group, C₄-C₂₀ cycloalkenyl group, and (C₃-C₁₀ cycloalkyl) C₁-C₁₀ alkyl group etc. When the hydrocarbyl group is used as an spacer in the present invention, the term refers to a divalent group formed by the removal of one hydrogen atom from one of the aforementioned groups.

In the present description the term "alkyl group" refers to an alkyl group that is either straight chain or branched, and includes, for example, a methyl, ethyl, propyl, n-butyl, tert-butyl, pentyl, and hexyl group, etc. Moreover, when the alkyl group is selected as A in the formula, in practice an alkylene group formed by the removal of one hydrogen atom from one of the aforementioned groups can be used as a spacer. Examples of the alkylene group include a methylene, ethylene, propylene, butylene, pentylene group, and hexylene group, etc.

In the present description the term "alkenyl group" refers to a straight chain or branched alkenyl group of 2-20 carbon atoms, preferably 2-10 carbon atoms, having 1 to 3 double bonds; more specifically, it includes ethenyl, 1-propenyl, 2-propenyl, 1-methyl ethenyl, 1-butenyl, 2-butenyl, 3-butenyl, 2-methyl-2-propenyl, 1-pentenyl, 2-pentenyl, 4-pentenyl, 3-methyl-2-butenyl, 1-hexenyl, 2-hexenyl, 1-heptenyl, 2-heptenyl, 1-octenyl, 2-octenyl, 1,3-octadienyl, 2-nonenyl, 1,3-nonadienyl, and 2-decenyl, etc.

The term "aryl group" includes, for example, a phenyl group, naphthyl group such as 1-naphthyl and 2-naphthyl, an indenyl group such as 2-indenyl, an anthryl group such as 2-anthryl, a tolyl group such as 2-tolyl, 3-tolyl and 4-tolyl, a biphenyl group, etc.

In the present description the term "heterohydrocarbyl group" refers to one of the aforementioned hydrocarbyl groups that also contains at least one heteroatom selected from a group consisting of nitrogen, oxygen, and sulfur, and it includes, for example, a C₁-C₂₀ straight chain or branched alkylene group in which one or more than one of the non-adjacent CH₂ groups is optionally substituted by - NH- , - NH- NH- , - NHCO- , - CONH- , - N(C₁₋₃ alkyl)-, -O-, -S- , - CO- , - O-CO-, -S- CO- , - O-COO-, -CO- S- , - CO-O-, - CH(halogen)-, - CH(CN), - CH=CH- , - NH- NH- CO- or - CO- NH- NH-.

Examples of groups that can serve as a substituent of the hydrocarbon group, heterocyclic group, etc., include a halogen atom (for example, fluorine, chlorine, bromide, and iodine, etc.), nitro group, cyano group, optionally halogenated C₁₋₆ alkyl group, etc.

In the present description, the term "biotinyl group" refers to any residue of biotin shown below, and in a narrow sense refers to a biotin residue shown below from which the hydroxyl group has been removed.

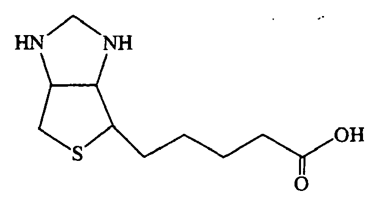

The biotin residue in the present invention may have any substituent provided it does not interfere with the purification, labeling etc., of the hemoprotein. Examples of such a substituent include a halogen atom (for example, fluorine, chlorine, bromide, and iodine, etc.), nitro group, cyano group, optionally halogenated C₁₋₆ alkyl group, etc.

### BRIEF DESCRIPTION OF THE DRAWINGS

Figure 1 shows the results of mass spectrography of the heme compound containing a biotinyl group that was obtained in Example 1;
Figure 2 shows the change in the ultraviolet-visible light (UV-Vis) absorption spectrum when the biotinyl heme was added to a cell extract containing the artificial heme protein dA1 in Example 2;
Figure 3 shows the electropherogram by SDS-PAGE of hemoprotein purified by the biotinyl heme obtained in Example 2; and
Figure 4 shows UV-Vis absorption spectrum of myoglobin reconstituted with the biotinyl heme in Example 3.

### BEST MODE FOR CARRYING OUT THE INVENTION

### (Preparing method)

The porphyrin compound containing a biotinyl group of the present invention can be synthesized by the method show in scheme (1) below, for example. 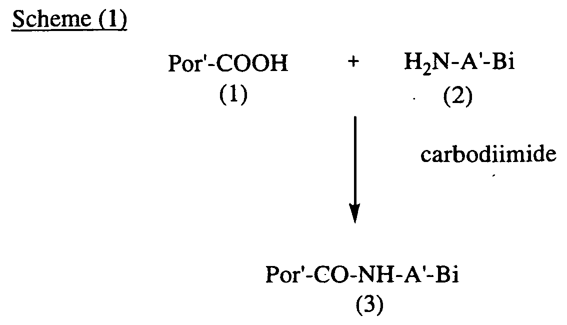
wherein Por' represents a residue wherein one carboxyl group has been removed from a porphyrin optionally forming a metal complex; A' represents a spacer group; and Bi represents a biotinyl group.

In scheme (1) above, compound 1 and a terminally aminated biotinyl compound 2 are reacted in the presence of a coupling agent such as a carbodimide, etc., to obtain the target porphyrin compound containing a biotinyl group 3. The terminally aminated biotinyl compound is preferably a hydrazidated biotinyl compound such as biotin hydrazide, 6-hydrazidohexyl-D-biotinamide, 6-(6-hydrazidohexyl) amidohexyl-D-biotinamide (which are well-known compounds on the market), etc. Preferably, this reaction is usually performed in the presence of a suitable solvent at 0°C-100°C, preferably 10°C-40°C, and for 0.5-48 hours and preferably 1-24 hours.

Herein, when a metal complex (for example, a heme compound containing a biotinyl group) is obtained as a final product, the metal complex of the porphyrin (for example, heme) can be reacted with the terminally aminated biotinyl compound in the presence of a coupling agent, or the porphyrin and the terminally aminated biotinyl compound may first be reacted in the presence of a coupling agent, followed by reaction with a metal (ion) to form the metal complex.

In scheme (I), the porphyrin 1 was illustrated as a model having one carboxyl group, but in practice a porphyrin may have a plurality of carboxyl groups. For example, the target heme compound of the present invention is preferably one in which a single biotinyl group is bonded to a heme. Therefore, it is necessary to adjust the amount of starting material used according to the number of carboxyl groups that the heme in question has. For example, iron protoporphyrin IX has two carboxyl groups. Therefore, if iron protoporphyrin IX is used as porphyrin 1, when 2 or more equivalents, and preferably 2.5 or more equivalents, of porphyrin 1 are used with respect to the hydrazidated biotinyl compound 2, a compound is obtained wherein the biotinylated compound is bonded to only one carboxyl group of the iron protoporphyrin IX via the hydrazide group.

For reference purposes to explain the mechanism of the above coupling reaction, scheme (2) shows an example wherein iron protoporphyrin IX and 6-hydrazidohexyl-D-biotinamide are reacted in the presence of a dicarboximide to obtain a porphyrin compound containing a biotinyl group.

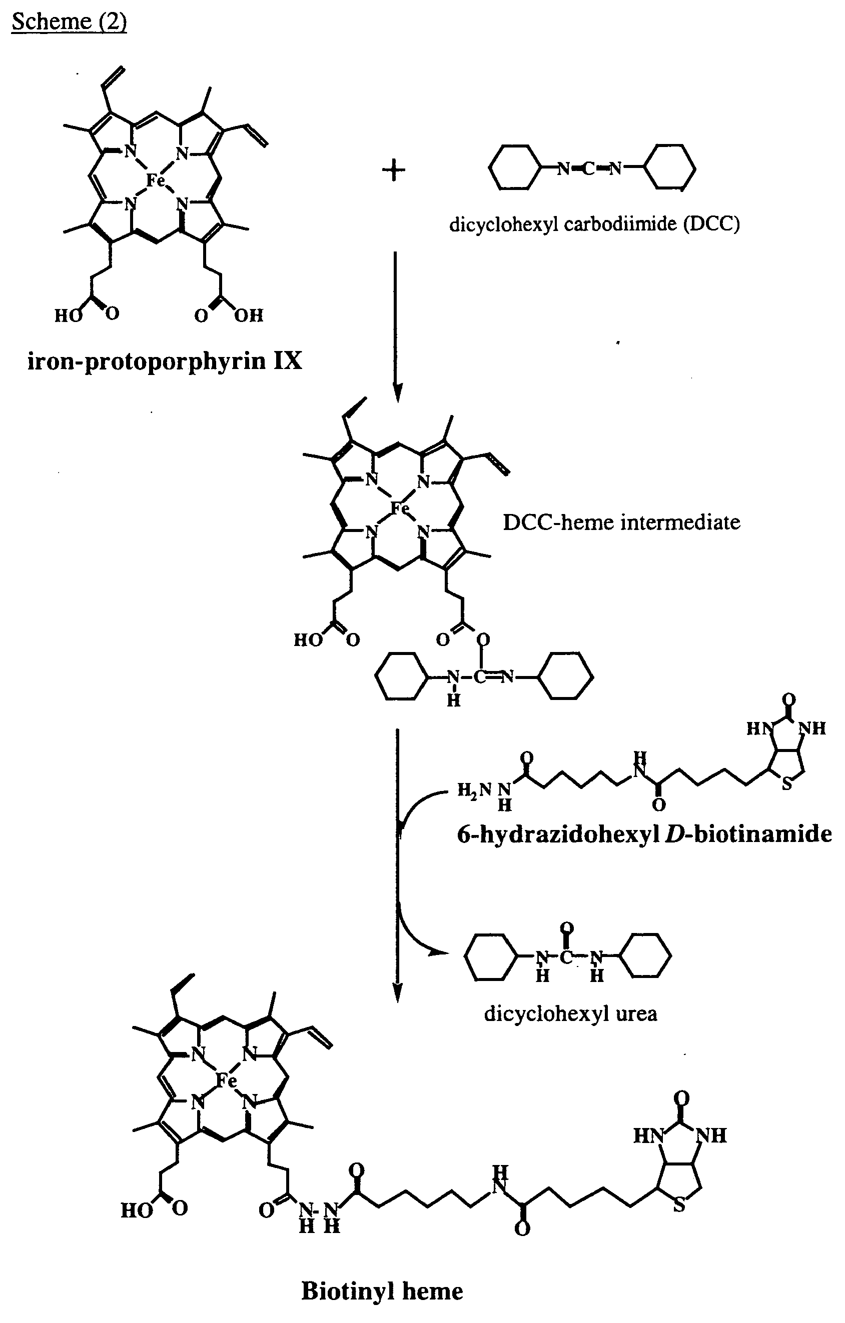

Examples of the coupling agent used in this reaction include the following: N,N'-dicyclohexyl carbodiimide (DCC), N'-(3-dimethylaminopropyl)-N-ethyl carbodiimide (DIC), 1-ethyl-3-(3-dimethylaminopropyl) carbodiimide, N-allyl-N'-(β-hydroxyethyl) carbodiimide, N-(a-dimethylaminopropyl)-N'-(β-bromo allyl) carbodiimide, 1-(3-dimethylaminopropyl)-3-(6-benzoyl aminohexyl) carbodiimide, cyclohexyl-β-(N-methyl morpholino) ethyl carbodiimide, ethyl-1,2-dihydro-2-ethoxy-1-quinolinecarboxylate (EEDQ), isobutyl-1,2-dihydro-2-isobutoxy-1-quinolinecarboxylate (IIDQ), 1-benzotriazolyloxy tris (dimethylamino)-phosphonium hexafluoro phosphate (HBTU), O-{[cyano-(ethoxy carbonyl)-methylidene]-amino}-1,1,3,3-tetramethyl uronium tetrafluoroborate (TOTU), propane phosphonic acid anhydride (PPA), 3-dimethylamino phosphinothioyl-2 (3H)-oxazolone (MPTO), etc.

The suitable solvent used in this reaction is not restricted provided the reaction proceeds, and it includes the following examples: aromatic amines such as pyridine, lutidine, and quinoline; halogenated hydrocarbons such as dichloromethane, chloroform, 1,2-dichloroethane, and carbon tetrachloride; aliphatic hydrocarbons such as hexane, pentane, and cyclohexane; aromatic hydrocarbons such as benzene, toluene, xylene, and chlorobenzene; ethers such as diethyl ether, diisopropyl ether, diphenyl ether, tetrahydrofuran, dioxane, and 1,2-dimethoxyethane; amides such as N,N-dimethylformamide and N,N-dimethylacetamide; as well as mixtures of two or more of the above solvents. Especially preferred solvents in the above reaction are dimethyl formamide (DMF), dimethyl sulfoxide (DMSO), or a mixture thereof.

If a "base" is used in the aforementioned reaction, it may be selected from the following examples: a basic salt such as sodium carbonate, potassium carbonate, and cesium carbonate; an inorganic base such as sodium hydroxide and potassium hydroxide; an aromatic amine such as pyridine and lutidine; a tertiary amine such as triethyl amine, tripropyl amine, tributyl amine, cyclohexyl dimethylamine, 4-dimethyl aminopyridine, N,N-dimethyl aniline, N-methyl piperidine, N-methyl pyrrolidine, and N-methyl morpholine; an alkali metal hydride such as sodium hydride and potassium hydride; a metal amide such as sodium amide, lithium diisopropyl amide, and lithium hexamethyl disilazide; and a metal alkoxide such as sodium methoxide, sodium ethoxide, and potassium tert-butoxide.

Isolation and purification of the end product obtained by the above reaction from the reaction mixture can be performed according to well-known means such as concentration, solvent extraction, fractional distillation, crystallization, recrystallization, and chromatography, etc.

For example, the above compound containing a terminally aminated biotinyl group 2 can be synthesized by the reaction shown in scheme (3) below.

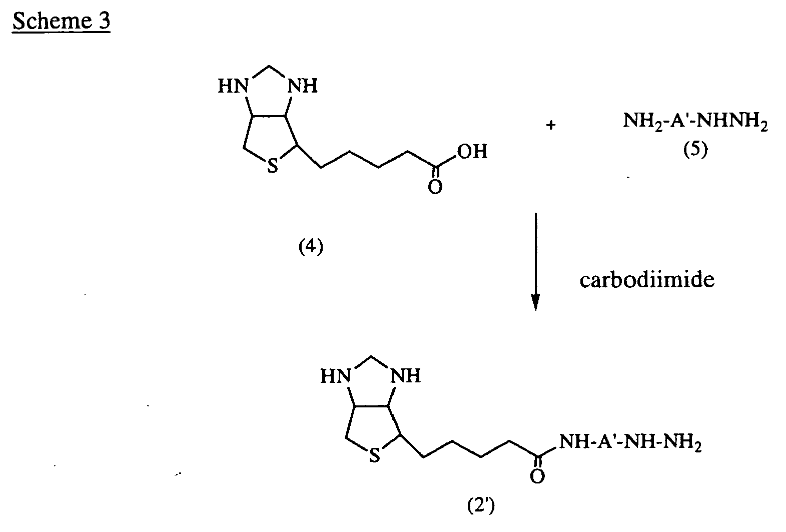

In scheme (3) above, the terminally aminated biotin compound 2' can be obtained by reacting biotin 4 and a hydrazide compound 5 in the presence of a coupling agent such as a carbodiimide, etc. In place of the hydrazide compound 5, it is possible to use a dihydrazide compound represented by the formula NH₂- NH- CO- A'- CO- NH- NH₂ or a diamine compound represented by the formula NH₂- A'- NH₂ (wherein A' represents a C₁-C₃₀ hydrocarbyl group or a C₁-C₃₀ heterocarbyl group having 1-6 heteroatoms selected from a group consisting of oxygen, sulfur and nitrogen). These reactions can be performed under the same reaction conditions as the coupling reaction of scheme (1).

In this manner it is possible to synthesize a compound in which both ends of the spacer group A in the formula Por-A-Bi are amino groups. Compounds in which the spacer group A is another group can be synthesized by ordinary persons skilled in the art using publicly known organic synthesis methods (see Bayer et al., Methods Biochem. Anal. 26 (1980), 1-45).

### (hemoprotein purification method)

The purification of the hemoprotein present in the samples is performed by using affinity chromatography with the above porphyrin compound containing a biotinyl group. As used herein, the term "affinity chromatography" refers to a method for isolating or purifying a target substance contained in a sample (for example, a body fluid sample such as serum, plasma, etc., or a culture supernatant, supernatant obtained by centrifugation, etc.) by utilizing the interactions (affinity) between specific substances such as antigen-antibody, enzyme-substrate, and ligand-receptor interactions. In the purification method of the present invention, isolation or purification of a heme-binding protein contained in the sample is performed by utilizing the specific affinity between the above porphyrin compound containing a biotinyl group and hemoprotein and the specific affinity of the biotinyl group and an avidin compound. The porphyrin compound of the present invention can purify hemoprotein present in a sample in a variety of embodiments using publicly known affinity chromatography techniques. For example, hemoprotein can be purified by a hemoprotein purification kit that contains the porphyrin compound of the present invention and carrier beads with an avidin compound bonded thereto.

In accordance with a preferred embodiment of the present invention, first the above porphyrin compound containing a biotinyl group is added to a sample containing the target hemoprotein, enabling the porphyrin compound to bind to the target hemoprotein. Next, an entity wherein an avidin compound such as avidin, etc., is bound to a carrier such as beads, etc., (hereinafter called "avidin beads") is added to the compound wherein the target hemoprotein is bound to the porphyrin compound (hereinafter called the "hemoprotein-porphyrin complex"), and utilizing avidin-biotin binding, the hemoprotein-porphyrin complex is bound to the avidin beads. Thus, the hemoprotein-porphyrin complex that is bound to the avidin beads can be recovered by publicly known means, and the target protein can be isolated and recovered by preparing a suspension in a solution containing a compound having the action of separating heme from protein such as imidazole, acid, guanidine hydrochloride or another denaturing agent, etc. The present invention places no particular restriction on the above carrier provided it is a carrier to which an avidin compound bonded thereto. For example, streptavidin magnetic beads, streptavidin agarose, etc., which are commercially available from Vector Laboratories and Pierce Biotechnology Inc. can be used. Moreover, using magnetized beads has the advantage that the collection can be performed more easily by magnet.

### (Hemoprotein labeling compound and applications thereof)

The porphyrin compound containing a biotinyl group that was obtained in the above manner can be used as a labeling compound for hemoprotein either alone or by bonding a labeling substance thereto. In this description, the term "labeling substance" means a substance used to facilitate detection of the presence thereofby physically or chemically bonding to the porphyrin compound containing a biotinyl group. More specifically, this term includes fluorescent substances such as fluorescein isothiocyanate, phycobiliprotein, a rare earth metal chelate, dansyl chloride or tetramethylrhodamine isothiocyanate bonded to an avidin compound such as avidin, streptavidin, etc.; or a radioactive isotope such as ³H, ¹⁴C, ¹²⁵I or ¹³¹I, etc. Among these possibilities, avidin compounds are most convenient because they are easy to obtain, and they can simply label the porphyrin compound containing a biotinyl group by utilizing avidin-biotin specific binding.

Hemoprotein contained in a sample (for example, a body fluid sample such as serum, plasma, etc., a culture supernatant or supernatant obtained by centrifugation) can be detected and quantified by publicly known technology using this kind of labeling compound or a diagnostic agent containing this compound. Moreover, the *in vivo* behavior, etc., of hemoprotein can be observed by using such a labeling compound. The diagnostic agent can be prepared in the form of a solution wherein the above compound is stably stored. It can be diagnosed whether a patient has a disease in which a specific hemoprotein is involved by comparing the detected amount of that specific hemoprotein present in the sample with the range of normal values. Heme oxygenase deficiency and leukemia involving a heme-associated transcription factor called Bach are known as such hemoprotein associated diseases. Moreover, the detection of human hemoglobin in feces caused by bleeding of the gastrointestinal organs (occult blood in stool) has been widely used as a method of testing for diseases of the digestive system such as colon cancer in recent years, and the diagnostic method of the present invention can be used to diagnose such a colon cancer.

### (Therapeutic drug for photodynamic therapy (PDT)

The porphyrin compound containing a biotinyl group of the present invention can be used as a therapeutic drug for PDT. When this porphyrin compound (active ingredient) is used as a therapeutic drug for PDT, it is mixed with a pharmacologically acceptable carrier, excipient, and diluent, etc., and usually administered in the form of an injection.

The active ingredient in the pharmaceutical preparation will be included, for example, in 0.1 to 30 wt%, preferably 1 to 5 wt%. The dose of this therapeutic drug will differ depending on the symptoms, age, weight, etc., of the patient but, for example, the daily amount of the active ingredient administered should be 0.05 mg to 30 mg, preferably 0.05 mg to 5 mg, and more preferably 0.05 mg to 1 mg per 1 kg of body weight of the patient. The content of the active ingredient and the dose are not limited to the above range, and are to be properly adjusted according to the type of active ingredient, carrier, excipient, diluent, etc., to be used. When PDT treatment is performed, preferably the diseased tissue where the tumor exists will be labeled with avidin before administration of treatment. Such avidin labeling can be performed by using an antibody to the protein (a tumor marker, etc.) expressed specifically by the tumor cells. Then the injection containing the above active ingredient is administered to the diseased tissue. Thus, because of the high affinity between biotin and avidin, the necessary porphyrin compound can be efficiently delivered in a site specific manner to the diseased tissue. Subsequently, the diseased tissue is irradiated with light, and the lesion can be destroyed by activation of the porphyrin. The irradiating light has a suitable wave-length (for example, 600-790 nm) and intensity (for example, 1-50 J/cm²) for activating the porphyrin. The irradiation of light is performed, for example for 1 minute to 2 hours, preferably 10 to 600 minutes. If necessary, the irradiation of light can be performed by using an optical fiber, etc., inserted in a catheter.

### EXAMPLES

The present invention is explained more specifically below based on examples.

### Example 1: Synthesis of porphyrin compound containing a biotinyl group

Iron protoporphyrin IX chloride (hemin) used as an experimental material was purchased from Sigma. The 6-hydrazidohexyl-D-biotinamide used was purchased from Vector Laboratories.

First of all, hemin and 6-hydrazidohexyl-D-biotinamide were dissolved in dehydrate DMF and DMSO at 6.7 mM and 2.7 mM, respectively. 20 µL of the biotin hydrazide solution and 5.6 mg of dicyclohexyl carbodiimide (DCC) were added to 1 mL of hemin solution. The reaction mixture was gently shaken and incubated in the dark at room temperature for 3 hours. In order to conjugate only one of the two propionate groups of protoheme with the biotin hydrazide, approximately 2.5 equivalent excess amounts of hemin were used for the reaction.

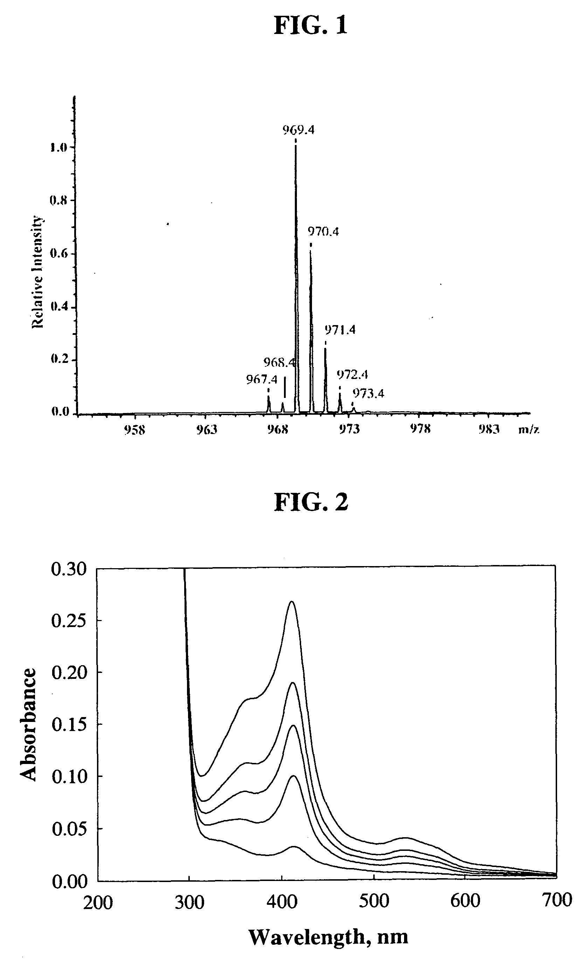

The reaction mixture made as above was supplement with approximately 5% (v/v) pyridine and was applied onto a C₁₈ reverse-phase preparative HPLC column COSMOSIL 5C₁₈-ARII (Nacalai Tesque). The porphyrin compound containing a biotinyl group (hereafter, referred to as "biotinyl heme") was eluted with a gradient of 40-60% acetonitrile in the presence of 0.1% TFA. The peak fraction containing the biotinyl heme was collected and immediately lyophilized in the dark. The sample was dissolved in the minimal volume of DMSO and stored at -80°C. The purity of the sample was examined using C₁₈ reverse-phase analytical HPLC column (COSMOSIL 5C₁₈-AR300, Nacalai Tesque). Identity of the purified molecule was verified by laser-desorption mass spectrometry (MALDI/TOFMS). Figure 1 shows the result of mass spectrometry.

This analysis indicated that the obtained compound had the mass of around 969.4 Da. This corresponds to the calculated mass of the biotinyl heme (969.98Da) in which one of the two propionate groups of the protoheme was conjugated with the biotin hydrazide. Therefore, the obtained compound was confirmed to be the biotinyl heme, which is the final compound shown in scheme (2) above.

### Example 2: Purification of heme protein using the biotinyl heme

Artificial genes encoding sperm whale myoglobin (Springer et al., Proc. Natl. Acad. Sci. USA 84 (1987), 8961-8965), designed globin-1 (DG1) (Isogai et al., Biochemistry 39 (2999), 5683-5690), and designed four-helix bundle hemoprotein (dA1) were cloned into a pRSET-C vector (Invitrogen). The amino acid sequence of dA1, (SEQ ID NO: 1, ML· KKLREEA · LKLLEEF · KKLLEEH · LKWLEGGGGGGGGELLKL · HEELLKK · FEELLKL · AEERLKK · L) was designed to form a four-helix bundle in the dimer and to bind one heme per monomer via bis-histidine ligation between the two helices according to the method of Gibney et al. [(Gibney et al., Biochemistry 37 (1988), 4635-4643). A synthetic gene encoding sperm whale myoglobin cloned into pUC19 vector (Springer et al., Proc. Natl. Acad. Sci. USA 84 (1987), 8961-8965) was also used to obtain a cell extract containing the native myoglobin. These hemoprotein-coding vectors were transformed into E. *coli* strain BL21 (DE3). For expression, Terrific Broth (liquid culture medium) supplemented with 100 mg/L ampicillin was grown under control of a T7 promoter using IPTG. Cells were harvested by centrifugation and were washed with 10 mM TRIS-HCl, pH 8.0 and 1 mM EDTA. The resultant pellets were suspended in a lysis buffer containing 6 M urea, 0.5 M NaCl, 1 mM EDTA and 0.1% ODP (octyl glucopyranoside) and were lysed by sonication. After removal of the insoluble fraction by centrifugation, the supernatant was collected and dialyzed with TN buffer. During these procedures, almost all heme associated with proteins in the cell extracts was removed and the proteins were refolded. After the insoluble fraction was removed by centrifugation, those proteins were concentrated to a suitable concentration using Centriprep-10 (Amicon). A cell extract obtained in the above manner was used as a starting material for the purification of a recombinant apohemoprotein using a biotinyl heme.

The biotinyl heme was added to the cell extracts obtained as mentioned above in small increments to finally 10 to 40 µM, and was incubated at 4°C for more than 30 minutes.

Figure 2 shows the changes in the UV-Vis absorption spectra when biotinyl heme was added to the cell extract containing the artificial hemoprotein dA1. In Figure 2, the lowest spectrum shows absorbance without the addition of biotinyl heme, and it is clear that the heme-bound dA1 concentration increases from bottom to top with the stepwise addition of biotinyl heme. The vertical axis shows the absorbance.

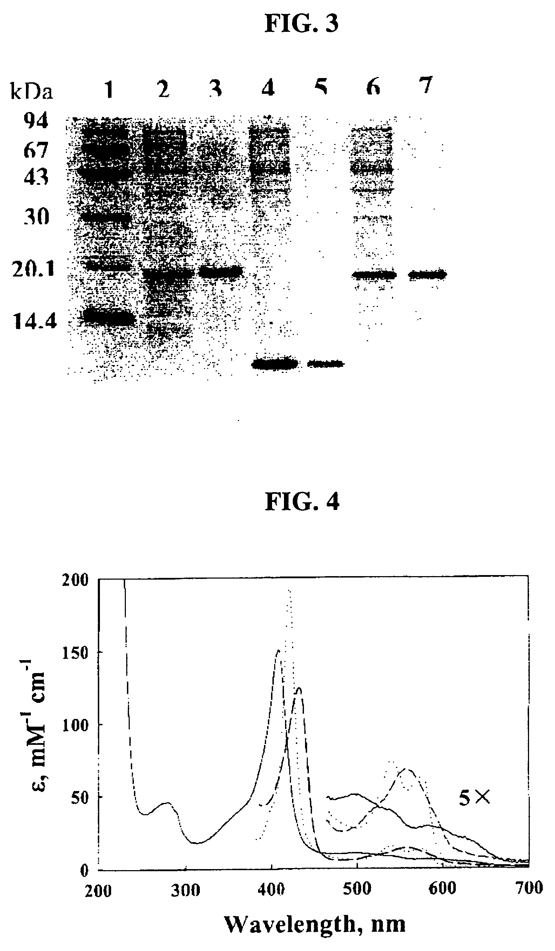

Next, after the removal of insoluble materials by centrifugation, the solutions were transferred to a sample tube containing streptavidin agarose (Sigma) or streptavidin magnetic beads (Pierce) pre-washed with a washing buffer containing 20 mM TRIS-HCl (pH 8.0), 500 mM NaCl, and 0.5% (v/v) Tween 20. The resultant protein-biotin-heme-streptavidin complexes were collected by centrifugation for the agarose complex or by using magnet for the magnetic bead complex. The pellets were washed twice with the washing buffer and incubated with 10 M imidazol (pH 8.0) to elute the bound proteins. The solution was desalted and lyophilized after removal of the agarose or magnetic beads. The lyophilized samples were dissolved in a small amount of TN buffer and were analyzed by SDS-PAGE with 15% (w/v) polyacrylamide gel. Figure 3 shows the SDS-PAGE electropherogram of the hemoprotein purified by the biotinyl heme. In Figure 3, lane 1 is the molecular size marker (from the top, 94, 67, 43, 30, 20.1, and 14.4 kDa); lanes 2 and 3 are the cell extract and the purified fraction of the recombinant myoglobin, respectively; lanes 4 and 5 are the cell extract and the purified fraction of dA1, respectively; and lanes 6 and 7 are the cell extract and the purified fraction of DG1, respectively.

As shown above, we have prepared three samples containing sperm whale myoglobin, designed globin-1 (DG1), and the designed four-helix bundle heme protein (dA1). Addition of the biotinyl heme into the cell extracts induced the intense Soret absorption bands characteristic of the bound heme in these proteins, indicating that it was effectively incorporated into the protein even in the dense mixture of biological molecules. From these mixtures, the reconstituted hemoproteins were easily collected by streptavidin magnetic beads without significant contamination of other proteins (see Fig. 3).

### Comparative example 1

After washing the magnetic beads in buffer, instead of adding the imidazole and eluting the apohemoprotein, the same protein was eluted by a process wherein an acid or a denaturing agent such as guanidine hydrochloride was added. In that instance, however, denatured streptavidin subunits that do not bind to the biotinyl heme nor to the beads coeluted with the hemoprotein. The protein was also purified using streptavidin agarose. However, the use of the agarose increased of the contamination due to non-specific interactions of protein with agarose.

In conclusion, it is clear that the biotinyl heme is a useful reagent to detection and purification of native and artificial heme protein. As described above, preparation of the biotinyl heme is simple and its specific ligation with native and artificial hemoproteins can be easily monitored with UV-Vis absorption spectroscopy.

### Example 3: Bonding of biotinyl heme to myoglobin(labeling)

Apomyoglobin was prepared from horse heart metmyoglobin using the methyl ethyl ketone extraction method described by Ascoli et al. (F. Ascoli, M.R. Fanelli, E. Antonini, Preparation and properties of apohemoglobin and reconstituted hemoglobins, Methods Enzymol. 76 (1981), 72-87). The heme-removed apoprotein was dialyzed against TN buffer containing 10 mM TRIS-HCl (pH 8.0) and 200 mM NaCl at 4°C. After removal of the insoluble fraction by centrifugation, the supernatant was concentrated to 1 to 2 mM with Centriprep 10 (Amicon). Reconstitution of myoglobin with the biotinyl heme was performed by addition of the biotin-heme solution into the apomyoglobin solution in increments of 0.1 to 0.2 equivalents to a small excess of the protein. This mixture was incubated for more than 30 minutes at 4°C and was centrifuged at 20,000xg for 30 minutes. The reconstituted biotin-heme myoglobin was collected in the supernatant and preserved significant stability similar to that of natural metmyoglobin as judged by measurements of the UV-Vis absorption spectrum.

The reconstituted myoglobin with the biotinyl heme was diluted with TN buffer to 10 to 20 µM and the UV-Vis absorption spectra were recorded with a Hitachi U-3000 spectrophotometer using a quartz cuvette of 1.0 cm in path length. The ferric, ferrous deoxy and ferrous CO-bond forms were prepared for the spectroscopic measurements according to the above reference by Ascoli et al.

Figure 4 shows the UV-Vis absorption spectra of myoglobin reconstituted with the biotinyl heme with the ferric (solid line), ferrous deoxy (broken line) and ferrous CO-bond forms (dotted line). These spectra were indistinguishable from those of native myoglobin. In addition, it was confirmed that the biotin-hem bound myoglobin preserved stable O₂-binding ability. These results suggest that the biotinyl heme is incorporated in the heme pocket of myoglobin in the manner similar to normal protoheme in myoglobin.

### INDUSTRIAL APPLICABILITY

As described above, according to the present invention, the present invention can provide a heme compound containing a biotinyl group that enables the rapid and simple purification of small amounts of hemoprotein in the living body. In addition, the present invention can provide a method for the simple purification of hemoprotein using this kind of heme compound containing a biotinyl group. Furthermore, the present invention can provide a hemoprotein labeling reagent and a diagnostic agent for hemoprotein associated diseases that uses that reagent. Moreover, the present invention can provide a novel therapeutic drug for use with photodynamic therapy.

## Claims

1. A porphyrin compound containing a biotinyl group represented by Formula (I):
Por-A-Bi
wherein Por represents a porphyrin residue optionally forming a metal complex; Bi represents an optionally substituted biotinyl group; and A represents a C₁-C₃₀ hydrocarbyl group, or a C₁-C₃₀ heterohydrocarbyl group having 1-10 heteroatoms selected from a group consisting of oxygen, sulfur, and nitrogen.

2. The compound according to claim 1, wherein Por is a porphyrin residue that has formed a metal complex selected from a group consisting of heme a, heme b, heme c, variant heme c, heme d, heme d1, siroheme, and heme o.

3. The compound according to claim 1 or 2, wherein the Por is a heme b residue.

4. The compound according to claim 1, wherein the Por is a porphyrin residue selected from a group consisting of uroporphyrin-I, uroporphyrin-II, coproporphyrin-III, protoporphyrin-IX), and hematoporphyrin-IX.

5. The compound according to any of claims 1 to 4, wherein the Bi is a biotinyl group.

6. The compound according to any of claims 1 to 5, wherein the A is a straight chain or branched alkylene group of 1-20 carbon atoms, and one or more than one of the non-adjacent CH₂ groups of the alkylene group is optionally substituted by - NH- , - NH- NH- , - NHCO- , - CONH- , - N(C₁₋₃ alkyl)- , - O-, - S- , - CO- , - O- CO- , - S- CO- , - O- COO- , - CO-S- , - CO- O- , - CH(halogen)- , - CH(CN)- , - CH=CH- , - NH- NH- CO- or - CO- NH- NH- .

7. The compound of any of claims 1 to 6, wherein the A is selected from a group consisting of
- NH- NH- ,
- NH- NH- CO- (CH₂)ₙ- NH- ,
- NH- NH- CO- (CH₂)ₙ- NH- CO- (CH₂)ₙ- NH- ,
- NH- (CH₂)ₙ- NH- ,
- NH- NH- CO- (CH₂)ₙ- NH- ,
- NH- NH- CO- (CH₂)ₙ- CO- NH- NH- ,
- NH- (CH₂)ₙ- CO- NH- NH- , and
- NH(CH₂)ₙ- CO- NH- (CH₂)ₙ- CO- NH- NH-
in these formulae each n independently represents 1-10.

8. A method for preparing the porphyrin compound containing a biotinyl group according to claim 1, comprising reacting a porphyrin optionally forming a metal complex with a compound containing a terminally aminated biotinyl group in the presence of a coupling agent.

9. A hemoprotein purification method, comprising a step of performing affinity chromatography using the compound according to claim 1.

10. A hemoprotein purification kit, comprising the compound according to claim 1 and carrier beads with an avidin compound bonded thereto.

11. A hemoprotein labeling compound that is the compound according to claim 1.

12. A method for detecting hemoprotein using the labeling compound according to claim 11.

13. A diagnostic agent for hemoprotein-associated diseases, comprising the labeling compound according to claim 11.

14. A therapeutic drug for photodynamic therapy, comprising the compound according to claim 4.


SEQUENCE LISTING

<110> RIKEN

<120> Biothinyl group-containing porphyrin compound and its use

<130> G05-0079

<140> PCT/JP2004/005645
<141> 2004-04-20

<150> 2003-116232
<151> 2003-04-21

<160> 1

<170> PatentIn Ver. 2.1

<210> 1
<211> 63
<212> PRT
<213> Artificial Sequence

<220>
<223> Description of Artificial Sequence: Synthetic peptide

<400> 1
Met Leu Lys Lys Leu Arg Glu Glu Ala Leu Lys Leu Leu Glu Glu Phe
1               5                   10                  15

Lys Lys Leu Leu Glu Glu His Leu Lys Trp Leu Glu Gly Gly Gly Gly
            20                  25                  30
Gly Gly Gly Gly Glu Leu Leu Lys Leu His Glu Glu Leu Leu Lys Lys
        35                  40                  45
Phe Glu Glu Leu Leu Lys Leu Ala Glu Glu Arg Leu Lys Lys Leu
    50                  55                  60